# FASCICULE DE BREVET EUROPEEN

(11) **EP 2 490 646 B1**
(45) Date de publication et mention de la délivrance du brevet: **04.09.2013**
(21) Numéro de dépôt: 10785148.7
(22) Date de dépôt: 20.10.2010
(51) Int. Cl.: B05B 11/00, B65D 83/14, A61M 15/08

(54) **TETE DE DISTRIBUTION POUR DISPOSITIF DE DISTRIBUTION DE PRODUIT FLUIDE.**
SPENDERKOPF FÜR EINEN FLUIDSPENDER
DISPENSING HEAD FOR A FLUID DISPENSER

(30) Priorité: 20.10.2009 FR 0957327
(43) Date de publication de la demande: 29.08.2012
(73) Titulaire: Aptar France SAS, 27110 Le Neubourg (FR)
(72) Inventeur: PARDONGE, Jean-Marc, F-76520 Les Authieux Sur Port Saint Ouen (FR)
(74) Mandataire: CAPRI
(86) Numéro de dépôt international: PCT/FR2010/052237
(87) Numéro de publication internationale: WO 2011/048332

(56) Documents cités:
- EP-A1- 1 380 351
- WO-A1-01/83010
- DE-A1- 2 830 977
- FR-A1- 2 736 285
- FR-A1- 2 771 296
- JP-A- 1 215 364
- US-A- 5 681 468
- US-B1- 6 264 936

## Description

La présente invention concerne une tête de distribution de produit fluide, et un dispositif de distribution de produit fluide comportant une telle tête de distribution.

Dans le domaine pharmaceutique, pour limiter la contamination du produit fluide notamment lors de sa distribution à travers la tête de distribution, il est connu d'appliquer des agents bactéricides et/ou bactériostatiques sur certaines surfaces en contact avec le produit fluide lors de sa distribution. Il a ainsi été proposé de prévoir de tels agents bactéricides et/ou bactériostatiques sur tout ou partie du chemin d'expulsion de la dose, depuis l'entrée du tube plongeur à travers le passage dans la pompe et jusque dans la tête de distribution, notamment au niveau de son orifice de distribution à travers lequel le produit sera distribué. Certains dispositifs comportent de plus des obturateurs disposés à l'intérieur de la tête de distribution directement en amont de l'orifice de distribution. Néanmoins, après chaque utilisation d'un tel dispositif, il peut rester une goutte résiduelle en aval de l'orifice de distribution, et notamment dans la petite cuvette formée autour de cet orifice par les parois de la tête de distribution. Cette goutte résiduelle est particulièrement susceptible d'être contaminée et elle est notamment susceptible d'être entraînée lors de l'utilisation suivante du dispositif avec donc un risque de contamination de la dose suivante. D'autre part, une tête de distribution nasale étant insérée à l'intérieur du nez de l'utilisateur lors de l'administration du spray, une partie importante de la surface de ladite tête peut entrer en contact avec les parois du nez et donc être contaminée. De nombreuses tentatives ont été menées pour conférer des propriétés bactériostatiques ou bactéricides aux matériaux plastiques utilisés pour la fabrication des têtes de distribution. Parmi ces solutions, il a été proposé de mouler des matériaux plastiques incluant de tels agents bactériostatiques dans la masse, par exemple des sels d'argent ou similaires, ou alors de traiter après moulage les surfaces, notamment par des revêtements de surfaces avec des couches contenant des agents bactéricides. Malheureusement, à ce jour aucune solution ne s'est avérée véritablement satisfaisante. Les documents FR 2 736 285 et US 6 264 936 décrivent des dispositifs de l'art antérieur.

La présente invention a pour but de fournir une tête de distribution et un dispositif de distribution de produit fluide comportant une telle tête qui ne reproduisent pas les inconvénients susmentionnés.

Plus particulièrement, la présente invention a pour but de fournir une tête de distribution fournissant une décontamination efficace de la goutte résiduelle qui est susceptible de rester à la sortie de l'orifice de distribution après chaque actionnement.

La présente invention a aussi pour but de fournir une tête de distribution nasale fournissant une décontamination efficace des surfaces susceptibles d'être contaminées lors de son insertion dans le nez d'un utilisateur.

La présente invention a aussi pour but de fournir une telle tête de distribution qui n'implique pas de modification du matériau plastique classiquement utilisé pour la réalisation des têtes de distribution

La présente invention a encore pour but de fournir une telle tête de distribution qui soit simple et peu coûteuse à fabriquer et à assembler.

La présente invention a donc pour objet une tête de distribution de produit fluide selon la revendication 1.

Avantageusement, ledit film est une feuille extrudée.

Avantageusement, ledit film a une épaisseur inférieure à 1 mm, avantageusement inférieure à 0,5 mm.

Avantageusement, ladite tête de distribution est surmoulée sur ledit film.

Avantageusement, ledit film est fixé, notamment déposé à chaud, thermo rétracté, soudé et/ou collé, à ladite tête de distribution, à l'extérieur et/ou à l'intérieur dudit orifice de distribution.

Avantageusement, ledit film est en forme de disque troué en son centre.

Avantageusement, ledit film définit l'orifice de distribution.

La présente invention a également pour objet un dispositif de distribution de produit fluide, comportant un réservoir contenant du produit fluide, un organe de distribution, tel qu'une pompe ou une valve, monté sur ledit réservoir, et comportant une tête de distribution telle que décrite ci-dessus.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique en section transversale d'un dispositif de distribution de produit fluide auquel la présente invention s'applique,
- la figure 2 est une vue schématique de détail d'une partie de la tête de distribution selon un mode de réalisation avantageux de la présente invention,
- la figure 3 est une vue schématique de coté d'une tête de distribution selon un autre mode de réalisation de l'invention, et
- la figure 4 est une vue de détail en section transversale de la figure 3.

Les techniques utilisées depuis de nombreuses années pour traiter les surfaces en contact avec le produit fluide à distribuer avec des agents bactéricides et/ou bactériostatiques, et notamment l'inclusion de tels agents dans la masse du matériau plastique ou le revêtement des surfaces après moulage avec de tels agents, se sont avérées insatisfaisantes. Des recherches ont démontré que des agents bactéricides et/ou bactériostatiques présentent une efficacité maximale lorsqu'ils sont incorporés dans un film de faible épaisseur, typiquement de l'ordre de quelques dixièmes de mm, et notamment d'une épaisseur inférieure à 1 mm, de préférence inférieure à 0,5 mm. Des tests en laboratoire ont démontré que lorsqu'ils sont effectués sur des films plastiques de faible épaisseur de ce type, les traitements bactéricides et/ou bactériostatiques s'avèrent nettement plus efficaces que lorsqu'ils sont appliqués sur des surfaces plus épaisses, typiquement sur une tête de pulvérisation d'un dispositif de distribution de produit fluide.

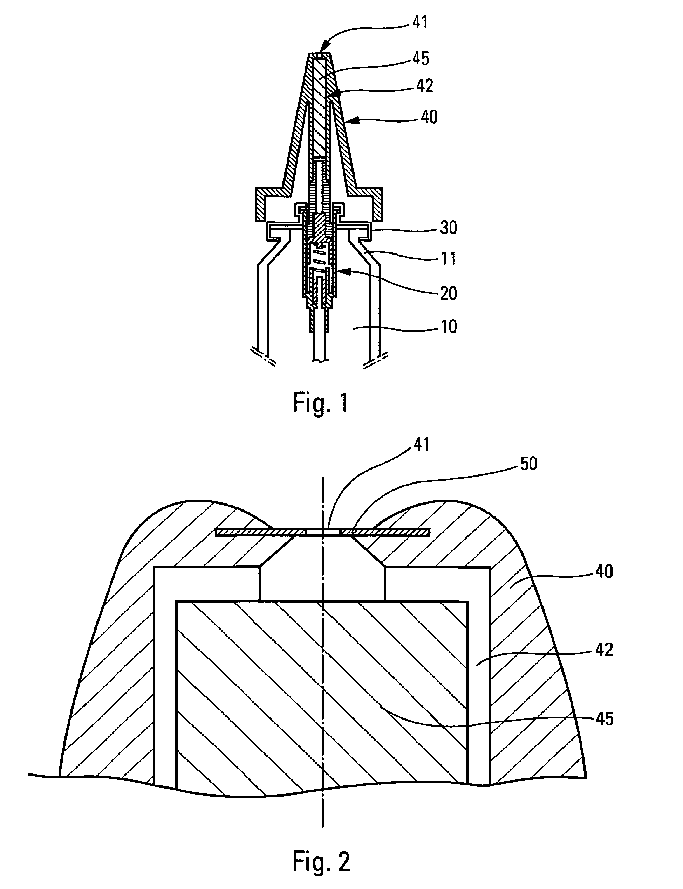

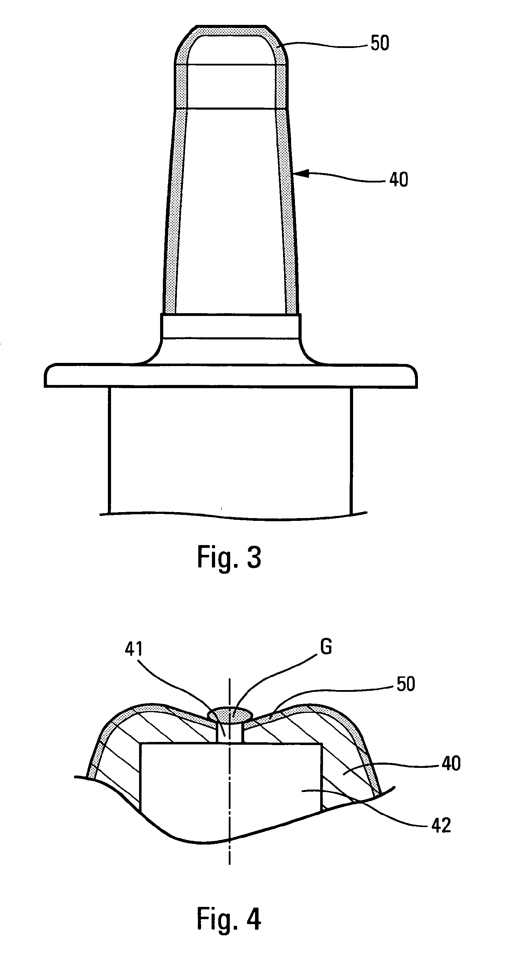

Selon l'invention, la présente invention prévoit donc de disposer un film 50 incorporant des agents bactéricides et/ou bactériostatiques au moins à proximité de l'orifice de distribution 41 d'une tête de distribution 40. Une telle tête de distribution 40 peut typiquement être une tête de distribution nasale comme schématiquement représenté sur les figures 1 et 3, montée sur une pompe ou une valve 20 elle-même fixée sur un réservoir 10 contenant le produit fluide à distribuer, par exemple au moyen d'une bague de fixation 30 qui fixe la pompe sur le col 11 du réservoir 10. La tête de distribution 40 peut typiquement comporter un orifice de distribution axial 41 prévu à l'extrémité avale d'une extension axiale destinée à pénétrer dans la narine de l'utilisateur, un canal d'expulsion 42 étant défini à l'intérieur de ladite tête 40 et un gicleur interne ou insert 45 étant prévu dans ledit canal d'expulsion 42 pour en limiter le volume mort et donc fournir un canal d'expulsion de faible dimension adapté à fournir une bonne pulvérisation du produit fluide à travers l'orifice de distribution 41. Un profil de pulvérisation (non représenté) peut classiquement être prévu au fond de la tête de distribution 40, directement en amont de l'orifice de distribution 41.

Comme visible sur la figure 2, qui montre une variante de réalisation de l'invention, un film 50 incorporant des agents bactéricides et/ou bactériostatiques est disposé au niveau de l'orifice de distribution 41. Dans l'exemple de la figure 2, la tête de distribution 40 est surmoulée autour dudit film 50, qui est ici réalisé sous la forme d'une feuille extrudée en forme de disque trouée en son centre. Dans cette variante de réalisation, c'est le film 50 lui-même qui définit l'orifice de distribution 41 proprement dit. Ainsi, la goutte résiduelle G qui est susceptible de stagner à l'extérieur dudit orifice de distribution 41 sera en contact avec ledit film 50, de sorte que les agents bactéricides et/ou bactériostatiques contenus dans ledit film 50 agiront efficacement sur ladite goutte résiduelle, limitant ainsi une contamination de la dose suivante. Bien entendu, la variante de réalisation représentée sur la figure 2 n'est qu'un exemple, et d'autres variantes sont envisageables. Par exemple, le disque pourrait être formé par une plaque rigide incorporant les agents bactéricides et/ou bactériostatiques.

Les figures 3 et 4 représentent un autre mode de réalisation de l'invention. Ici, le film 50 est appliqué sur l'extérieur de la tête de distribution. Dans le cas d'une tête nasale, telle que représentée sur la figure 3, le film 50 est appliqué au moins sur la partie de la tête qui pénètre dans la narine de l'utilisateur, à savoir l'extension axiale qui incorpore à son extrémité l'orifice de distribution 41. Avantageusement, le film mince 50 est disposé dans un moule approprié, et la matière plastique formant la tête de distribution est injectée à l'intérieur dudit film. En variante, le film mince peut être conformé et/ou fixé à la tête de distribution par tout autre moyen, par exemple déposé à chaud, thermo rétracté, soudé et/ou collé sur la tête de distribution, pour envelopper la forme de la tête. Eventuellement, le film mince peut aussi s'étendre à l'intérieur à l'extérieur dudit orifice de distribution 41. Tout autre moyen pour appliquer ou fixer un tel film mince incorporant des agents bactéricides et/ou bactériostatiques au niveau de l'orifice de distribution 41 de la tête de distribution 40 est aussi envisageable. La caractéristique importante est ici que la goutte résiduelle G susceptible de rester après chaque utilisation à l'extérieur de l'orifice de distribution 41 soit en contact avec un film mince incorporant de tels agents bactéricides et/ou bactériostatiques. De plus, dans le cas d'une tête de distribution nasale, les résidus de contact avec la narine éventuellement présents sur l'extérieur de la tête sont aussi traités.

L'invention présente en outre l'avantage de ne pas avoir à modifier les matériaux plastiques utilisés pour mouler la tête de distribution de produit fluide. Or, de manière connue, toute modification d'un matériau dans le cadre d'un dispositif de distribution de produit fluide dans le domaine pharmaceutique est compliquée et donc coûteuse, puisqu'elle doit être agréée par les différentes instances médicales et de santé des différents pays. La présente invention permet de garder les mêmes matériaux plastiques tout en garantissant un traitement efficace de la goutte résiduelle tel que décrit ci-dessus. De même, le fait de limiter le contact entre un matériau incorporant des agents bactéricides et/ou bactériostatiques et le produit fluide à l'endroit où c'est réellement nécessaire limite les échanges entre le produit fluide à distribuer et les agents bactéricides et/ou bactériostatiques, comme cela peut être le cas lorsque la totalité ou une grande partie des surfaces en contact avec ledit produit fluide sur le chemin d'expulsion de doses sont traitées.

Bien que la présente invention ait été décrit par référence à deux modes de réalisation particuliers de celle-ci, il est entendu qu'elle n'est pas limitée à ces modes de réalisation, mais qu'au contraire un homme du métier peut y apporter toutes modifications utiles sans sortir du cadre de la présente invention tel que défini par les revendications annexées.

## Revendications

1. Tête de distribution de produit fluide (40) adaptée à être assemblé sur un réservoir (10) contenant ledit produit fluide pour sélectivement distribuer ledit produit fluide au moyen d'un organe de distribution (20), tel qu'une pompe ou une valve, actionnée par ladite tête de distribution (40), ladite tête de distribution (40) comportant un orifice de distribution (41), **caractérisée en ce que** ladite tête de distribution (40) comporte un film (50) incorporant des agents bactéricides et/ou bactériostatiques, ledit film (50) étant disposé à proximité dudit orifice de distribution (41), ladite tête de distribution (40) étant une tête de distribution nasale destinée à être insérée dans une narine d'un utilisateur, ledit film (50) s'étendant à l'extérieur de ladite tête de distribution (40) au moins sur la partie pénétrant à l'intérieur de la narine.

2. Tête selon la revendication 1, dans laquelle ledit film (50) est une feuille extrudée.

3. Tête selon la revendication 1 ou 2, dans laquelle ledit film (50) a une épaisseur inférieure à 1 mm, avantageusement inférieure à 0,5 mm.

4. Tête selon l'une quelconque des revendications précédentes, dans laquelle ladite tête de distribution (40) est surmoulée sur ledit film (50).

5. Tête selon l'une quelconque des revendications précédentes, dans laquelle ledit film (50) est fixé, notamment déposé à chaud, thermo rétracté, soudé et/ou collé, à ladite tête de distribution (40), à l'extérieur et/ou à l'intérieur dudit orifice de distribution (41).

6. Tête selon l'une quelconque des revendications précédentes, dans laquelle ledit film (50) est en forme de disque troué en son centre.

7. Tête selon l'une quelconque des revendications précédentes, dans laquelle ledit film (50) définit l'orifice de distribution (41).

8. Dispositif de distribution de produit fluide, comportant un réservoir (10) contenant du produit fluide, un organe de distribution (20), tel qu'une pompe ou une valve, monté sur ledit réservoir, **caractérisé en ce qu'**il comporte une tête de distribution (40) selon l'une quelconque des revendications précédentes.

## Patentansprüche

1. Ausgabekopf für ein fluides Produkt (40), der dafür geeignet ist, auf einem Behälter (10) angebracht zu werden, der das fluide Produkt enthält, um das fluide Produkt selektiv mittels einer Ausgabevorrichtung (20), wie einer Pumpe oder einem Ventil, auszugeben, die durch den Ausgabekopf (40) betätigt wird, wobei der Ausgabekopf (40) eine Ausgabeöffnung (41) aufweist, **dadurch gekennzeichnet, dass** der Ausgabekopf (40) einen Film (50) aufweist, der bakterizide und/oder bakteriostatische Mittel umfasst, wobei der Film (50) in der Nähe der Ausgabeöffnung (41) angeordnet ist, wobei der Ausgabekopf (40) ein nasaler Ausgabekopf ist, der dafür gedacht ist, in ein Nasenloch eines Benutzers eingeführt zu werden, wobei sich der Film (50) zumindest über den Teil, der ins Innere des Nasenloches eindringt, außerhalb des Ausgabekopfes (40) erstreckt.

2. Kopf nach Anspruch 1, wobei der Film (50) eine extrudierte Folie ist.

3. Kopf nach Anspruch 1 oder 2, wobei der Film (50) eine Dicke von weniger als 1 mm, vorteilhafterweise weniger als 0,5 mm aufweist.

4. Kopf nach einem der vorhergehenden Ansprüche, wobei der Ausgabekopf (40) auf den Film (50) aufgegossen ist.

5. Kopf nach einem der vorhergehenden Ansprüche, wobei der Film (50) auf den Ausgabekopf (40) an der Außenseite und/oder der Innenseite der Ausgabeöffnung (41) fixiert, insbesondere thermisch aufgebracht, thermisch eingezogen, geschweißt und/oder geklebt ist.

6. Kopf nach einem der vorhergehenden Ansprüche, wobei der Film (50) in Form einer Scheibe mit einem Loch in der Mitte vorliegt.

7. Kopf nach einem der vorhergehenden Ansprüche, wobei der Film (50) die Ausgabeöffnung (41) definiert.

8. Ausgabevorrichtung für ein fluides Produkt, aufweisend einen Behälter (10), der das fluide Produkt enthält, eine Ausgabevorrichtung (20), wie eine Pumpe oder ein Ventil, die auf dem Behälter montiert ist, **dadurch gekennzeichnet, dass** sie einen Ausgabekopf (40) nach einem der vorhergehenden Ansprüche aufweist.

## Claims

1. A fluid dispenser head (40) that is adapted to be assembled on a reservoir (10) containing said fluid, so as to dispense said fluid selectively by means of a dispenser member (20), such as a pump or a valve, that is actuated by said dispenser head (40), said dispenser head (40) including a dispenser orifice (41), said dispenser head (40) being **characterized in that** it includes a film (50) that incorporates bactericidal and/or bacteriostatic agents, said film (50) being arranged in the proximity of said dispenser orifice (41), said dispenser head (40) being a nasal dispenser head for inserting into a user's nostril, said film (50) extending over the outside of said dispenser head (40), at least over the portion that penetrates into the nostril.

2. A head according to claim 1, wherein said film (50) is an extruded sheet.

3. A head according to claim 1 or claim 2, wherein said film (50) has a thickness that is less than 1 mm, advantageously less than 0.5 mm.

4. A head according to any preceding claim, wherein said dispenser head (40) is molded on said film (50).

5. A head according to any preceding claim, wherein said film (50) is fastened, in particular hot deposited, shrink-fitted, heat-sealed, and/or adhesively-bonded, to said dispenser head (40), outside and/or inside said dispenser orifice (41).

6. A head according to any preceding claim, wherein said film (50) is in the shape of a disk, having a hole at its center.

7. A head according to any preceding claim, wherein said film (50) defines the dispenser orifice (41).

8. A fluid dispenser device comprising: a reservoir (10) containing fluid; and a dispenser member (20), such as a pump or a valve, mounted on said reservoir; the device being **characterized in that** it further includes a dispenser head (40) according to any preceding claim.
